(19) Europäisches Patentamt
European Patent Office
Office européen des brevets

(11) EP 4 166 166 A1

(12) EUROPEAN PATENT APPLICATION

(43) Date of publication:
**19.04.2023 Bulletin 2023/16**

(21) Application number: **21202928.4**

(22) Date of filing: **15.10.2021**

(51) International Patent Classification (IPC):
***A61L 15/46*** *(2006.01)* ***D06M 13/265*** *(2006.01)*
***A61L 15/24*** *(2006.01)* ***C08L 33/08*** *(2006.01)*

(52) Cooperative Patent Classification (CPC):
(C-Sets available)
**A61L 15/46; A61L 15/24; C08L 33/08;**
**D06M 13/265;** A61L 2300/202; A61L 2300/204;
A61L 2300/216 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Mölnlycke Health Care AB**
**402 52 Göteborg (SE)**

(72) Inventors:
- **Elias, Milja**
**44694 Skepplanda, Ale (SE)**
- **Wellner, Eric**
**41574 Göteborg (SE)**

(74) Representative: **Tostmann, Holger Carl**
**Wallinger Ricker Schlotter Tostmann**
**Patent- und Rechtsanwälte Partnerschaft mbB**
**Zweibrückenstrasse 5-7**
**80331 München (DE)**

(54) **FUNCTIONALIZED POLYMER PARTICLES OF FIBERS FOR ODOR CONTROL IN WOUND CARE**

(57) The present invention relates to polymer particles or polymer fibers covalently bonded to N-chloroamines, N,N-dichloroamines, N-chloro sulfonamides or N,N-dichloro sulfonamides, for removing volatile organic compounds (VOCs) from a space above a wound. The removal of said VOCs is believed to be primarily or predominantly by chemical reaction of the VOCs with the N-chloro or the N,N dichloro group as covalently attached to the polymer. In particular, the functionalized polymer particles or polymer fibers are part of a wound dressing and have the functionality to control, in particular reduce, odor emanating from wounds, without interacting with the wound. The dressings according to the present invention can be advantageously used, in particular, in the treatment of chronic wounds or infected wounds.

EP 4 166 166 A1

(52) Cooperative Patent Classification (CPC): (Cont.)

C-Sets
**A61L 15/24, C08L 29/04;**
**A61L 15/24, C08L 33/08**

**Description**

Field of the Invention

The present invention relates to polymer particles or polymer fibers covalently bonded to N-chloroamines, N,N-dichloroamines, N-chloro sulfonamides or N,N-dichloro sulfonamides, for removing volatile organic compounds (VOCs) from a space above a wound, wherein the removal of said VOCs is primarily or predominantly by chemical reaction of the VOCs with the chloro-group as immobilized on the polymer.

In particular, the "active chlorine"-functionalized polymer particles or polymer fibers are part of a wound dressing and have the functionality to control, in particular reduce, odor emanating from wounds, without interacting with the wound, in particular without significantly or noticeably leaching into the wound. The dressings of the present invention can be advantageously used, in particular, in the treatment of chronic wounds or infected wounds.

In other aspects, the present invention relates to a method of controlling the odor above a wound space, said method including a step of providing the functionalized polymer particles or polymer fibers of the present invention.

Background of the Invention

As disclosed, for example, in the review article "A Comprehensive Review of Topical Odor-Controlling Treatment Options for Chronic Wounds" by A. Akhmetova et al., J Wound Ostomy Continence Nurs. (2016), 43(6), pages 598 to 609, various methods or products are available to manage wound odor. One of the more commonly used compositions for odor control over wounds is activated charcoal (which may be part of wound dressing). Charcoal is generally understood to absorb or "trap" VOCs due to the large active surface area of (activated) charcoal. No chemical reaction is believed to be involved in this physical adsorption mechanism.

In other approaches, silver or iodine are used as components in wound dressings, for example as coatings on a non-woven material or as impregnated in viscose rayon. Silver is believed to *indirectly* reduce odor by way of its antimicrobial properties. In that sense, silver should rather be seen as an antimicrobial agent (and as such may be part of the wound dressing of the present invention) than an odor reducing agent. Similarly, iodine is used to reduce the bacterial burden in the wound bed and therefore also reduces odor by way of disrupting bacterial wall membranes.

In an alternative approach, metronidazole (which is commonly used as a topically applied antibiotic) is used for the management of wound odor because of its ability to reduce odor producing anaerobic pathogens in selected wounds.

Natural compounds are also known as means to control wound malodor, for example honey or essential oils.

None of these approaches (silver, iodine, antibiotics, "natural" antibacterial products) are based on a mechanism of chemically reacting with the VOCs produced by bacteria/microbes/germs.

While all the known approaches as described above have their advantages, they are also associated with certain limitations. For example, the problem with wound control measures that involve physical adsorption, in particular charcoal, is the potential of saturation as the VOCs are not converted into non-odorous substances but are *"stored"* until capacity is reached. Furthermore, indirect methods that reduce or kill bacteria are not suitable for all wound types and only deal with odor due to bacterial infection. Such methods may not be suitable for all wounds, in particular not for chronic wounds.

Furthermore, many of these known approaches require that the (antimicrobial) agent penetrates into the wound in order to be effective, i.e. the agent is indeed an active ingredient that requires full clinical studies and certification.

Other sources of volatile organic compounds are metabolites produced by humans and secreted via the skin. Also, necrotic tissue as commonly present in chronic wounds may release VOCs.

A known approach to address odors in hygiene articles (e.g. in diapers) is the use of sulfonamides. As an example, US 8,425,890 describes halo active aromatic sulfonamides for use in controlling odor in bodily fluids in personal hygiene articles such as diapers and sanitary napkins. According to the prior art, the sulfonamides, for example the halo active aromatic sulfonamides according to US 8,425,890 are used as *such,* i.e. are used as chemical compounds that are added (e.g. coated onto) the overall product. For example, in US 8,425, 890, wood fluff is treated with an aqueous solution of the sulfonamide compounds.

Simply spraying chemical compounds onto a substrate (and then enclosing the substrate inside the article) may be a suitable approach for hygiene articles that are meant to take up bodily fluids secreted from the human body. However, the situation is different for wound dressings since wound dressings come in immediate contact with wound beds typically comprising open or compromised blood vessels, which means that the entry of chemically active compounds into the wound is generally not desirable or may even be detrimental to wound healing. Therefore, wound dressings that comprise active compounds that may leach out of the dressing are not desirable and the concept disclosed in US 8,425, 890 cannot be readily transferred to wound dressings.

Summary of the Invention

Overall, in view of the above, one object of the present invention is to provide means for controlling odor, in particular odor above wounds, wherein said means avoid or mitigate any or all of the disadvantages mentioned above.

In particular, an object of the present invention is to provide odor control means for wounds, in particular for chronic wounds, that do not expose the wound to direct contact with reactive chemical compounds and that permanently remove volatile organic compounds irrespective of their source.

This problem and others is/are solved, in a first aspect, by polymer particles or polymer fibers that are covalently bonded to N-chloroamines, or N,N-dichloroamines or covalently bonded to N-chloro or N,N-dichloro sulfonamides, wherein said functionalized polymer fibers or particles are used for removing volatile organic compounds, wherein the moiety as bonded to the polymer particles or polymer fibers is of a structure selected from the following:

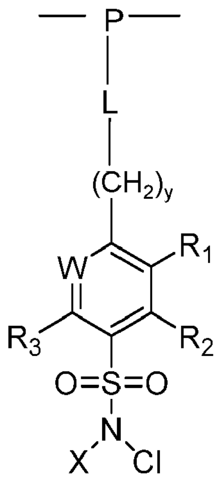
,
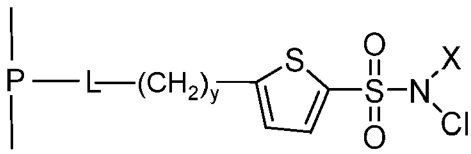
,

$$P\text{-}L\text{-}(CH_2)_y\text{-}SO\text{-}NXCl$$

or

$$P\text{-}L\text{-}(CH_2)_y\text{-}NXCl,$$

wherein:

P- is a polymer;

L is a linker;

y is an integer greater than zero;

W is N or -CH;

$R_1$ , $R_2$ and $R_3$ are independently selected from -H-, -alkyl, or —halogen;

X is Na, H or Cl.

Without wishing to be bound by theory, the chlorine group of the moiety that is covalently bonded to the polymer is believed to remove VOCs primarily or predominantly by chemical reaction, i.e. the VOCs are cleaved into non (or less) odorous reaction products.

In embodiments, one of the following, preferably all of the following applies:

$L\text{-}(CH_2)_y$ is $-NH-(CH_2)_y$; and/or

y is from 1 to 20, preferably from 1 to 12 or from1 to 6 and/or

$R_1$ is selected from -H-, $-CH_3$-, or —Cl-; and/or

halogen is F, Cl or Br and/or:
$R_2$ and $R_3$ are -H-.

In embodiments particularly relating to the claimed N-chloro sulfonamides or N,N-dichloro sulfonamides, y is from 1 to 3

In embodiments particularly relating to the claimed N-chloroamines or N,N-dichloroamines, y is from 2 to 12

In embodiments, the polymer of the polymer particles or polymer fibers is based on polyacrylic acid, partially hydrolyzed acetyl-poly(vinylalcohol) or poly(vinyl alcohol), optionally a cross-linked polymer network of polyacrylic acid, partially hydrolyzed acetyl-poly(vinylalcohol) or poly(vinyl alcohol), or any copolymer or mixture thereof, wherein polyacrylic acids are preferred.

In embodiments, at least 0.1% of the alcohol or carboxylic acid groups originally present in the polymer are functionalized with a N-chloro or N,N--dichloro unit, preferably at least 0.5%.

In embodiments, the fraction of modified of polymer, i.e. degree of functionalization of the alcohol or carboxylic acid groups originally present in the polymer with a N-chloro or N,N--dichloro unit, is from 0.1% to 80%, preferably from 0.5% to 50%.

In embodiments, the amount of active chlorine as determined by the method provided in the experimental section below is from 0.1% to 20% (w/w), optionally from 0.3% to 16% (w/w).

In embodiments, the polymer particles are superabsorbent polymer particles (SAPs).

In accordance with the present invention and in accordance with the IUPAC definition provided in Pure Appl. Chem., Vol. 76, No. 4, pp. 889-906, 2004, a superabsorbent polymer is a polymer that can absorb and retain extremely large amounts of liquid relative to its own mass, preferably 10 times its weight or 100 times its weight.

In embodiments, the polymer fibers are superabsorbent fibers (SAFs).

In embodiments the median $D^{50}$ particle diameter of the polymer particles is from 80 $\mu$m to 600 $\mu$m, optionally from 150 $\mu$m to 400 $\mu$m; or wherein the average diameter of the fibers is from 1 $\mu$m to 300 $\mu$m, optionally from 5 $\mu$m to 100 $\mu$m.

In a second aspect, the above problem is / the above problems are solved by a wound dressing comprising the polymer particles or polymer fiber as described herein.

In embodiments, the fibers according to the present invention are preferentially incorporated in an airlaid fiber structure while the particles according to the present invention are preferentially incorporated in a foam.

In embodiments, at least 5% by weight, relative to the overall weight, optionally at least 10% by weight of the wound dressing is provided by the polymer particles or polymer fiber as described herein.

In embodiments, the polymer particles or polymer fibers as described herein have a grammage of at least 15 $g/m^2$, optionally at least 30 $g/m^2$ of the overall wound dressing.

In embodiments, the wound dressing furthermore comprises at least one of the following:
(a) a backing layer; (b) at least one absorbent layer; (c) a wound contact layer.

In embodiments, said wound contact layer comprises or consists of a silicone gel.

In embodiments, the polymer particles or polymer fibers according to the present invention are provided as part of the absorbent layer.

In embodiments, the absorbent layer comprises superabsorbent particles and/or superabsorbent fibers *not* functionalized with N-chloro or N,N-dichloro groups, i.e. SAPs and/or SAFs, the primary function of which is to absorb wound exudate.

In embodiments, the wound dressing comprises superabsorbent particles and/or superabsorbent fibers *not* functionalized with N-chloro or N,N-dichloro sulfonamides. Wherein, in addition, polymer particles or polymer fibers functionalized with N-chloro or N,N-dichloro groups are provided *separately* from said superabsorbent particles or superabsorbent fibers not functionalized with N-chloro or N,N-dichloro groups.

In embodiments, the polymer particles or polymer fibers functionalized with N-chloro or N,N-dichloro groups are provided in a layer farther away from the area of the wound dressing that is intended to be put in contact with the wound (wound contact layer) relative to the layer that comprises the superabsorbent particles or superabsorbent fibers not functionalized with N-chloro or N,N-dichloro groups. This spatial separation ensures that those SAPs or SAFs that have the primary functionality of absorbing wound exudate are indeed close to the wound, while those SAPs and SAFs that are functionalized to cleave VOCs are removed away from the wound.

In a third aspect, the above-mentioned problem(s) is/are solved by a method of controlling odor above a wound space, said method comprising at least the following steps:

(a) providing polymer particles or polymer fibers as described above and herein in a wound dressing;
(b) bringing at least a fraction of said polymer particles or polymer fibers into contact with at least one volatile organic

compound that is exuded from a wound and/or from the human skin in the vicinity of a wound;
(c) chemically reacting said volatile organic compound with N-chloro or N,N-dichloro groups as covalently bonded to said polymer particles or polymer fibers thus at least partially reducing odor emanating from a wound or from the area around a wound.

In embodiments, said at least one volatile organic compound is selected from heteroarylic, arylic compounds, sulfides, di-sulfides, trisulfides, ketones, alcohols, aldehydes, amines or carboxylic acids and esters.
In embodiments, said at least one volatile organic compound is selected from electron rich heteroarylic and arylic compounds.
In embodiments, said at least one volatile organic compound is selected from indole, 3-methyl indole, pyrimidine, acetophenone, isovaleric acid, 2,5-dimethylpyrazine, pyrrole, p-cresol, 2-aminoacetophenone, 6-methyl-5-heptene-2-one, cadaverine.
In embodiments, the wound is selected from chronic wounds (in contrast to "acute" wounds) and/or infected wounds.

**Brief Description of the Figures**

**Figure** 1 shows a synthesis scheme for functionalizing polymer particles or fibers with mono- or dichloro sulfonamide groups (1) and with mono or dichloro-amine groups (2). The synthesis is described in more detail below in the Experimental Section.
In this figure, EDC-HCl is N-(3-Dimethylaminopropyl)-N'-ethylcarbodiimide hydrochloride, NHS is N-Hydroxy-succinimide, MES is 4-Morpholineethanesulfonic acid monohydrate, HCl is Hydrochloric acid, $NaHCO_3$ is Sodium bicarbonate, NaOCl is sodium hypochlorite and HAc is Acetic acid.

**Figure** 2 shows SEM images of A) non-functionalized superabsorbent fibers (SAFs) B) N-chloro-sulfonamide modified SAF and C) N,N-dichloro-sulfonamide modified SAF.
As can be taken from the scanning electron microscopy pictures, the diameter of unmodified SAF is around 30μm whereas the diameter of N-chloro-sulfonamide modified SAF is around 40μm and that of N,N-dichloro-sulfonamide modified SAF around 50μm.

**Figure** 3 shows that superabsorbent particles (here: polyacrylic acid, these particles are also referred to as "PAA") and superabsorbent fibers SAF (also based on polyacrylic acid) as *such,* i.e. *without being modified* in accordance with the present invention essentially do not affect (reduce) the amount of volatile organic compounds. In these Figures and in the following Figures the VOCs are: Indole, 3-Methyl indole (3MeInd), Pyrimidine (Pyrim), Acetophenone (APh), Isovaleric acid (Isoval), 2,5-Dimethylpyrazine (2,5-Dimepy), Pyrrole, p-Cresol, 2-aminoacetophenone (2-AAph), 6-Methyl-5-heptene-2-one (6M-hept-one) and Cadaverine (CAD). The one exception to the general observation that the unmodified polymers do not reduce the amount of VOCs is that superabsorbent fibers as such indeed do reduce (or even remove) cadaverine (see lower panel of Figure 3).

**Figures 4 to** 7 show how functionalization of SAPs and SAFs with mono- or dichloro sulfonamide groups or with mono or dichloro-amine groups leads to a significant to essentially complete removal of a large range of VOCs.

Figure 4, upper panel, shows the effect of functionalized SAF polymer 2 as described in the experimental section below on selected VOCs while Figure 4, lower panel, shows the effect of functionalized SAP Polymer 1 as described in the experimental section below on selected VOCs

Figure 5, upper panel, shows the effect of different amounts of functionalized SAP Polymer 2 as described in the experimental section below on selected VOCs while Figure 5, lower panel, shows the effect of functionalized SAF Polymer 3 as described in the experimental section below on selected VOCs

Figure 6, upper panel, shows the effect of functionalized SAF polymer 5 as described in the experimental section below on selected VOCs while Figure 6, lower panel, shows the effect of functionalized SAF Polymer 4 as described in the experimental section below on selected VOCs

Figure 7, upper panel, shows the effect of functionalized SAF polymer 6 as described in the experimental section below on selected VOCs while Figure 7, lower panel, shows the effect of functionalized SAF Polymer 7 as

described in the experimental section below on selected VOCs

**Figures 8 and** 9 show tables providing values for the % reduction of VOCs for functionalized polymers as described in the Experimental Section below.

## Detailed Description of Embodiments of the Invention

One advantage of the present invention is that the volatile organic compounds are not simply absorbed but that an actual chemical reaction takes place that converts the odorous VOCs into non-odorous or otherwise harmless smaller molecules. Since a chemical reaction takes place, there is, in principle, no limitation to the amount of VOCs that can be removed from a wound space or the vicinity of a wound space by the functionalized polymer particles or fibers as described herein.

Also, importantly, due to immobilization of the chemically reactive moiety with the N-chloro or the N,N dichloro group to a polymer backbone, release of a water soluble active component into the wound is avoided or at least substantially avoided.

This puts the present invention in contrast to existing odor control measures in hygiene articles, wherein sulfonamides are not truly immobilized or not at all immobilized, for example merely sprayed or coated onto a substrate and thus may be easily released into a fluid system and thus may leach into the wound, which is generally undesirable (see "Background" section)

A further advantage of the present invention is that polymer particles or polymer fibers that are already commonly used in wound dressings, for example superabsorbent polymers or superabsorbent fibers can be functionalized to carry the herein disclosed N-chloro or the N,N dichloro group

In embodiments, superabsorbent polymer particles or superabsorbent fibers are partially functionalized with the N-chloro or the N,N dichloro group as disclosed herein thus leading to superabsorbent particles or superabsorbent fibers that are still superabsorbent but that also have VOCs cleaving functionality.

In accordance with the present invention "partially functionalized" means that 5% to 75% of all available OH-groups of a particle or fiber are functionalized, preferably 15% to 60%.

In other embodiments, polymer fibers or polymer particles are fully or essentially fully functionalized with N-chloro or the N,N dichloro groups as disclosed herein and such fully or essentially fully functionalized superabsorbent fibers or superabsorbent particles are used *together* with the same or different superabsorbent polymer particles or superabsorbent polymer fibers that are not functionalized or are only partially functionalized and thus primarily function as superabsorbent materials.

In other embodiments, several layers are provided in a wound dressing, wherein the layer closer to the wound space comprises superabsorbent particles or superabsorbent fibers that are not functionalized or only partially functionalized with N-chloro or N,N dichloro groups while a second, separate layer of superabsorbent particles or superabsorbent fibers is provided farther away from the wound space, which then comprises fully essentially fully functionalized superabsorbent polymers or superabsorbent fibers having the primary functionality to cleave VOCs.

A further advantage of the present invention is that due to the mechanism of chemically reacting with VOCs, i.e. *directly* removing the VOCs, the mechanism that leads to VOCs or the origin of the VOCs is irrelevant and wounds can be treated in which the origin of the VOCs is not primarily of bacterial origin.

An additional benefit of the sulfonamide functionalized polymer particles or polymer fibers is that in use, i.e. in contact with wound exudate, the claimed and disclosed N-chloro or N,N dichloro group are capable of releasing hypochlorite which is known to have antimicrobial properties and can thus also contribute to the lowering of the bioburden in a dressing and/or in the wound.

## Examples (Experimental)

**1.) Synthesis** (see Figure 1)

Reagents:

Polyacrylic acid (PAA) - Mol. Wt. - 1,250,000 Da - Sigma Aldrich

SAF - Super absorbent fiber - Technical Absorbents

AEBSA - 4-(2-Aminoethyl)benzenesulfonamide, 99% - Thermo Fisher Scientific

MES - 4-Morpholineethanesulfonic acid monohydrate - Thermo Scientific™ BupH MES Buffered Saline Pack

NaOCl - sodium hypochlorite (6-14% active chlorine) - Honeywell Fluka

HAc - glacial acetic acid - Sigma Aldrich

N-Boc-ethylenediamine - Sigma Aldrich

N-Boc-1,6-hexanediamine - Sigma Aldrich

Synthesis of polvacrvlic acid polymer particles functionalized with N-chloro/N,N-dichloro sulfonamide

Step 1 - EDC coupling

1g PAA was added to 65 mL MES buffer (0.05M, pH=6.0) in a 250 mL RB flask. Then 0.760g EDC-HCl and 0.920g NHS dissolved in 15 mL MES buffer (0.05M, pH=6.0) was added. This was stirred at RT for 30 minutes. Then 1.2g AEBSA dissolved in 20 mL phosphate buffer solution (0.1M, pH=7.0) was added and the reaction continued at RT for 4h. After 4h, the sulfonamide bound polymer was washed with deionized water to remove all the unreacted reagents and impurities. The formed product was then dried at RT.

Step 2 - Chlorination

**Polymer 1-** 1g of the sulfonamide bound polymer was taken in a round bottom flask and added deionized water to swell the polymer. Then 16mL of NaOCl solution and 1mL HAc was added. The reaction was continued in an ice bath for 4h. The formed product was washed with large amount of deionised water till the washings did not show presence of chlorine (Absence of blue color with KI and starch solution).

Synthesis of polymer fibers functionalized with N-chloro/N,N-dichloro sulfonamides

Step 1- EDC coupling

1g SAF was added to a 250 mL RB flask followed by 20 mL of MES buffer. 0.76g EDC and 0.92g NHS was dissolved in 8 mL of MES buffer and added to SAF in MES buffer. After stirring for 30 minutes, 1.2g AEBSA dissolved in 20 mL of phosphate buffer (pH= 7.0) was added. Stirred for 4h. The product was washed several times with deionised water and dried at room temperature.

Step 2 - Chlorination

**Polymer 2** - 1g of sulfonamide bound superabsorbent fibers (SAF) was added to 250mL RB flask. Approx.120mL deionised water was added to swell the fiber. Then 16mL NaOCl and 1mL gl. acetic acid was added and the reaction continued for 4h in an ice bath. The formed product was washed with large amount of deionised water till the washings did not show presence of chlorine (Absence of blue color with KI and starch solution). Dried at room temperature.

**Polymer 3** - 1g of sulfonamide bound SAF was added to 250mL RB flask. Approx. 120mL deionised water was added to swell the fiber. Then 16mL NaOCl was added and the reaction continued for 4h in an ice bath. The formed product was washed with large amount of deionised water till the washings did not show presence of chlorine (Absence of blue color with KI and starch solution). Dried at room temperature.

Synthesis of polymer fibers functionalized with N-chloro/N,N-dichloroamines

Step 1- EDC coupling was carried out as mentioned above for SAF with N-Boc protected diamines instead of AEBSA.

Step 2- Deprotection:- 0.25 g of fibers modified with N-Boc protected amine was added to 10 mL 4MHCl and stirred at room temperature for 3h. This was filtered, added deionised water and neutralised with sodium bicarbonate. The formed amine modified fibers were filtered, washed with deionized water and dried at RT

Step 3 - Chlorination

**Polymer 4** - Same procedure was used as for polymer 2 to get N,N-dichloro- ethylene diamine modified SAF.

**Polymer 5** - Same procedure was used as for polymer 3 to get N-chloro-ethylene diamine modified SAF.

**Polymer 6** - Same procedure as for polymer 2 to get N,N-dichloro-hexamethylene diamine modified SAF.

**Polymer** 7 - Same procedure as for polymer 3 to get N-chloro-hexamethylene diamine modified SAF.

**2.) Determination of amount of active chlorine in the modified polymers-Iodometric titration**

0.5g of KI was added to 10 mL deionized water. 50 mg of the modified polymer was added followed by 3 drops of 1% starch solution as indicator. A blue color formed which disappears at end point upon titration with 0.1 N (for N,N-dichloro compounds) or 0.01 N (for N-chloro compounds) sodium thiosulphate solution. Titration was done in triplicates for all samples.

The amount of active chlorine was calculated using the following equation:

$$Cl^{+}(\%) = \frac{35.45XNXV}{2XW} \text{ X } 100$$

wherein N is the equivalent concentration (N) and V is the volume (L) of the sodium thiosulphate solution used for titration, W is the weight of the modified polymer (g).

The following amounts of active chlorine were determined using this titration method:

Polymer 1 - Polyacrylic acid modified with N,N-dichlorosulfonamide -4%
Polymer 2 - SAF modified with N,N-dichlorosulfonamide - -4% and -14%
Polymer 3 - SAF modified with N-chlorosulfonamide - ~ 2%
Polymer 4 - SAF modified with N,N-dichloro-ethylene diamine -8%
Polymer 5 - SAF modified with N-chloro-ethylene diamine - ~ 0.3 - -0.6%
Polymer 6 - SAF modified with N,N-dichloro-diaminohexane - ~ 8%
Polymer 7 - SAF modified with N-chloro-diaminohexane - ~ 1%

**3.) Testing reduction of VOC concentration with modified polymers**

VOCs emitted by bacteria commonly found in wound (P. aeruginosa, S. aureus, E. coli) were selected from literature. These were divided into two mixtures for ease of calibration, because of co-eluting peaks, to get good peak separation.

Mix 1:- Indole, 3-Methyl indole (3MeInd), Pyrimidine (Pyrim), Acetophenone (APh), Isovaleric acid (Isoval), 2,5-Dimethylpyrazine (2,5-Dimepy)

Mix 2:- Pyrrole, p-Cresol, 2-aminoacetophenone (2-AAph), 6-Methyl-5-heptene-2-one (6M-hept-one), Cadaverine (CAD).

Method:

➢ VOCs -A concentration in the validated range was chosen and the solutions were prepared as Mix 1 and Mix 2 in simulated wound fluid (SWF).

➢ Polymer material (polymer 1 to 7, 100 mg) was added to 2.5 mL each of Mix1 and Mix 2 solution in SWF. For polymer 2 the amount was varied as specified in Figure 8.

➢ Vortexed for one minute.

➢ Incubated with shaking at 35 deg C for 24h.

➢ Supernatant analysed by HPLC-MS.

The concentration of VOCs in SWF after incubation for 24h was measured and from that the percentage reduction after adding the materials was calculated.

**4.) Results**

As shown in Figure 3, the non-modified (non-functionalized) polymer particles or fibers do not affect the VOC concentration (with the exception of SAFs reducing cadaverine).

Figures 4 to 7 as well as the tables reproduced in Figure 8 and Figure 9 show that, depending on the specific modification (Polymers 1 to 7 as described above) essentially all of the exemplarily chosen VOCs can be removed by

way of chemical reaction. Figure 5 shows that at least VOCs can already be removed by a comparatively low amount of functionalized polymer.

Figures 4 to 9 provide evidence that the inventive concept works well for both polymer particles and fibers and that functionalization with chloroamines and with chlorosulfonamides is effective in reducing VOCs.

## Claims

1. Polymer particles or polymer fibers covalently bonded to N-chloroamines or N,N-dichloroamines or covalently bonded to N-chloro or N,N-dichloro sulfonamides, for removing volatile organic compounds, wherein the moiety as bonded to the polymer particles or polymer fibers is of a structure selected from the following:

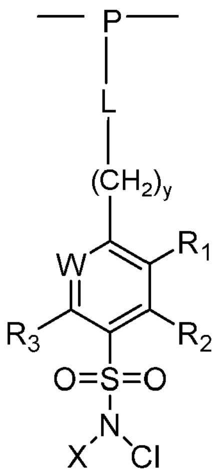
,
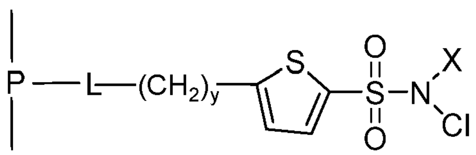
,

$P\text{-}L\text{-}(CH_2)_y\text{-}SO_2\text{-}NXCl$

or

$P\text{-}L\text{-}(CH_2)_y\text{-}NXCl$,

wherein:

P- is a polymer;
L is a linker;
y is an integer greater than zero
W is N or -CH;
$R_1$ , $R_2$ and $R_3$ are independently selected from -H-, -alkyl, or —halogen;
X is Na, H or Cl.

2. Polymer particles or polymer fibers of claim 1, wherein

$L\text{-}(CH_2)_y$ is $\text{-}NH\text{-}(CH_2)_y$; and/or
y is from 1 to 20, preferably from 1 to 12 or from 1 to 6 and/or
$R_1$ is selected from -H-, $\text{-}CH_3\text{-}$, or —Cl-; and/or
halogen is F, Cl or Br and/or:
$R_2$ and $R_3$ are -H-.

3. Polymer particles or polymer fibers of claim 1 or claim 2, wherein the polymer is based on polyacrylic acid, partially hydrolyzed acetyl-poly(vinylalcohol) or poly(vinyl alcohol), optionally a cross-linked polymer network of polyacrylic acid, partially hydrolyzed acetyl-poly(vinylalcohol) or poly(vinyl alcohol), or any copolymer or mixture thereof, preferably wherein the polymer is based on polyacrylic acid.

4. Polymer particles or polymer fibers according to any one of the preceding claims, wherein at least 0.1% of the alcohol

or carboxylic acid groups originally present in the polymer are functionalized with a N-chloro or N,N--dichloro unit, preferably at least 0.5%.

**5.** Polymer particles or polymer fibers according to any one of the preceding claims, wherein the amount of active chlorine as determined by the method provided in the description is from 0.1% to 20% (w/w), optionally from 0.3% to 15%.

**6.** Polymer particles or polymer fibers according to any one of the preceding claims, wherein the median D50 particle diameter of the polymer particles is from 80 μm to 600 μm, optionally from 150 μm to 400 μm; or wherein the average diameter of the fibers is from 1 μm to 300 μm, optionally from 5 μm to 100 μm.

**7.** Wound dressing, comprising the polymer particles or polymer fibers of any one of the preceding claims.

**8.** Wound dressing according to claim 7, comprising at least 5% by weight of said polymer particles or polymer fibers, relative to the overall weight of the dressing, optionally at least 10% by weight and/or wherein the polymer particles or polymer fibers of any one of the preceding claims have a grammage of at least 15 $g/m^2$, optionally at least 30 $g/m^2$ of the overall wound dressing.

**9.** Wound dressing according to claim 7, furthermore comprising at least one of the following:

(a) a backing layer;
(b) at least one absorbent layer;
(c) a wound contact layer, said wound contact layer optionally comprising a silicone gel.

**10.** Wound dressing according to claim 8 or claim 9, wherein the polymer particles or polymer fibers are provided as part of the absorbent layer, optionally wherein the absorbent layer comprises superabsorbent particles or superabsorbent fibers not functionalized with N-chloro or N,N-dichloro groups.

**11.** Wound dressing according to any one of claims 7 - 10, wherein the wound dressing also comprises superabsorbent particles or superabsorbent fibers not functionalized with N-chloro or N,N-dichloro sulfonamides. wherein the polymer particles or polymer fibers functionalized with N-chloro or N,N-dichloro sulfonamides are provided separately from the superabsorbent particles or superabsorbent fibers not functionalized with N-chloro or N,N-dichloro sulfonamides, in particular wherein the polymer particles or polymer fibers functionalized with N-chloro or N,N-dichloro sulfonamides are provided in a layer farther away from the area of the wound dressing that is intended to be put in contact with the wound than a layer that comprises the superabsorbent particles or superabsorbent fibers not functionalized with N-chloro or N,N-dichloro groups.

**12.** Method of controlling odor above a wound space, said method comprising at least the following steps:

(a) providing polymer particles or polymer fibers according to any one of claims 1 to 6 in a wound dressing;
(b) bringing at least a fraction of said polymer particles or polymer fibers into contact with at least one volatile organic compound that is exuded from a wound and/or from the human skin in the vicinity of a wound;
(c) chemically reacting said volatile organic compound with the N-chloro or N,N-dichloro group as covalently bonded to said polymer particles or polymer fibers thus at least partially reducing odor emanating from a wound or from the area around a wound.

**13.** Method according to claim 12, wherein said at least one volatile organic compound is selected from heteroarylic, arylic compounds, sulfides, di-sulfides, trisulfides, ketones, alcohols, aldehydes, amines or carboxylic acids and esters.

**14.** Method according to claim 12, wherein said at least one volatile organic compound is selected from indole, 3-methyl indole, pyrimidine, acetophenone, isovaleric acid, 2,5-dimethylpyrazine, pyrrole, p-cresol, 2-aminoacetophenone, 6-methyl-5-heptene-2-one, cadaverine.

**15.** Wound dressing or method according to any one of claims 1 to 14, wherein the wound is selected from chronic wounds and/or infected wounds.

## Figure 1

1) N-chloro or N,N-dichlorosulfonamide modified polymer

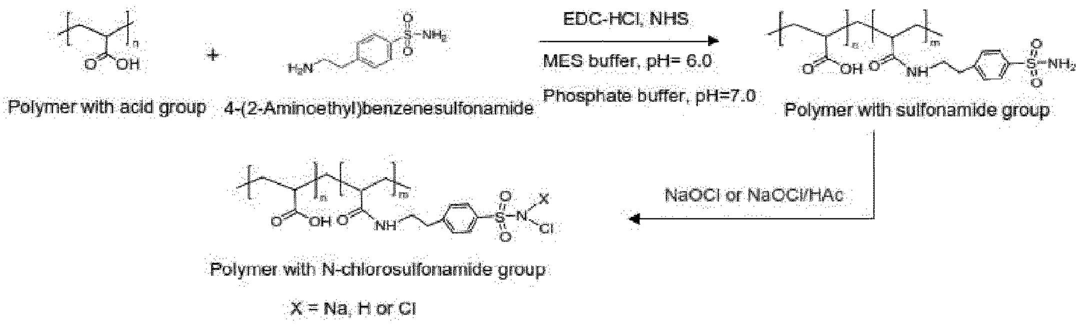


2) N-chloro or N,N-dichloroamine modified polymer

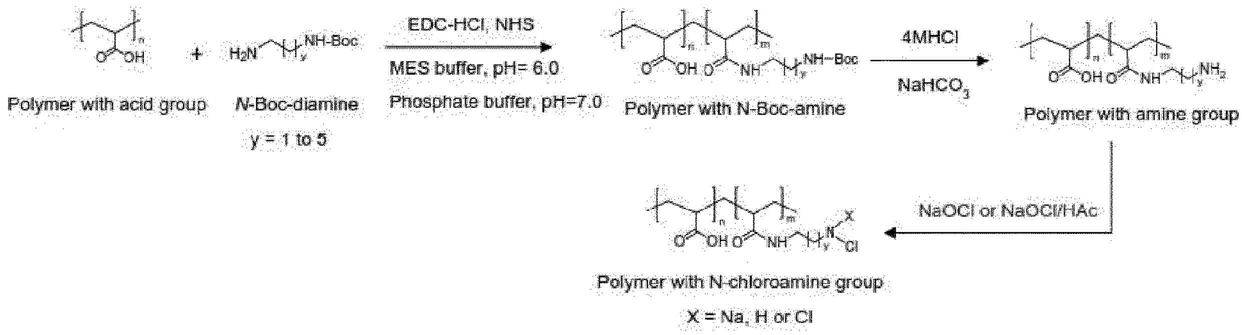

Figure 2:

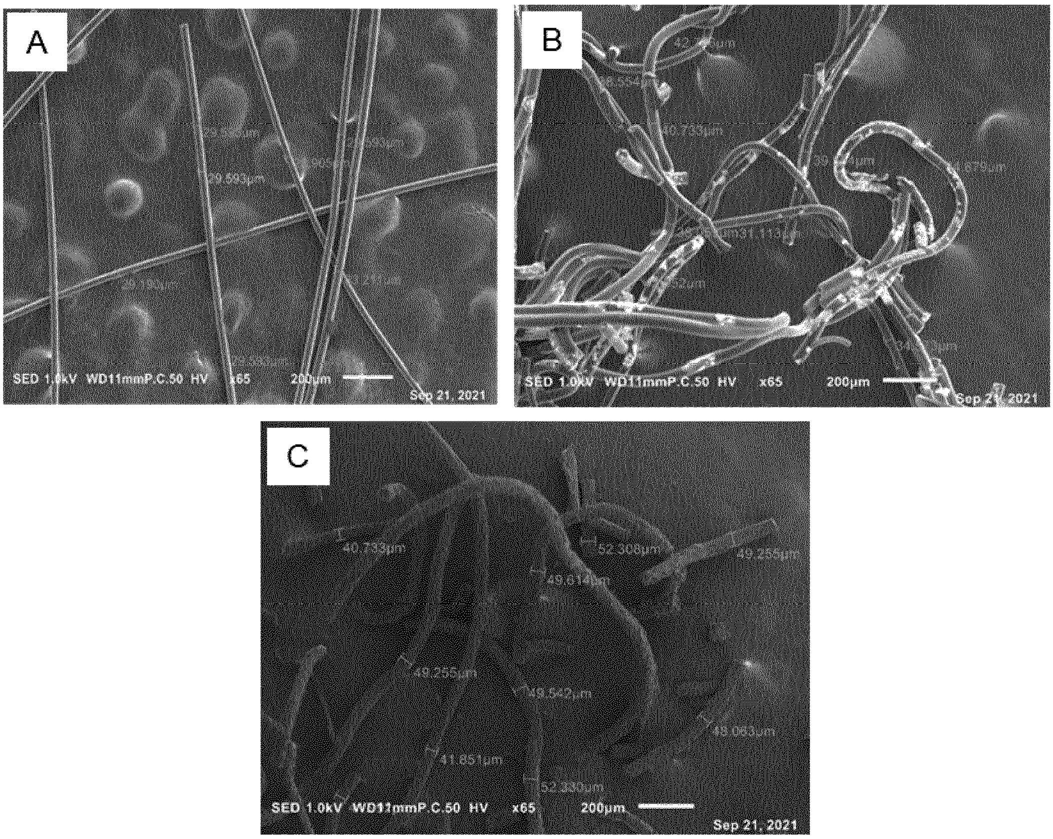
A
SED 1.0kV WD11mmP.C.50 HV x65 200µm Sep 21, 2021
B
SED 1.0kV WD11mmP.C.50 HV x65 200µm Sep 21, 2021
C
40.733µm
52.308µm
49.255µm
49.614µm
49.255µm
49.542µm
48.063µm
41.851µm
52.330µm
SED 1.0kV WD11mmP.C.50 HV x65 200µm Sep 21, 2021

Figure 3

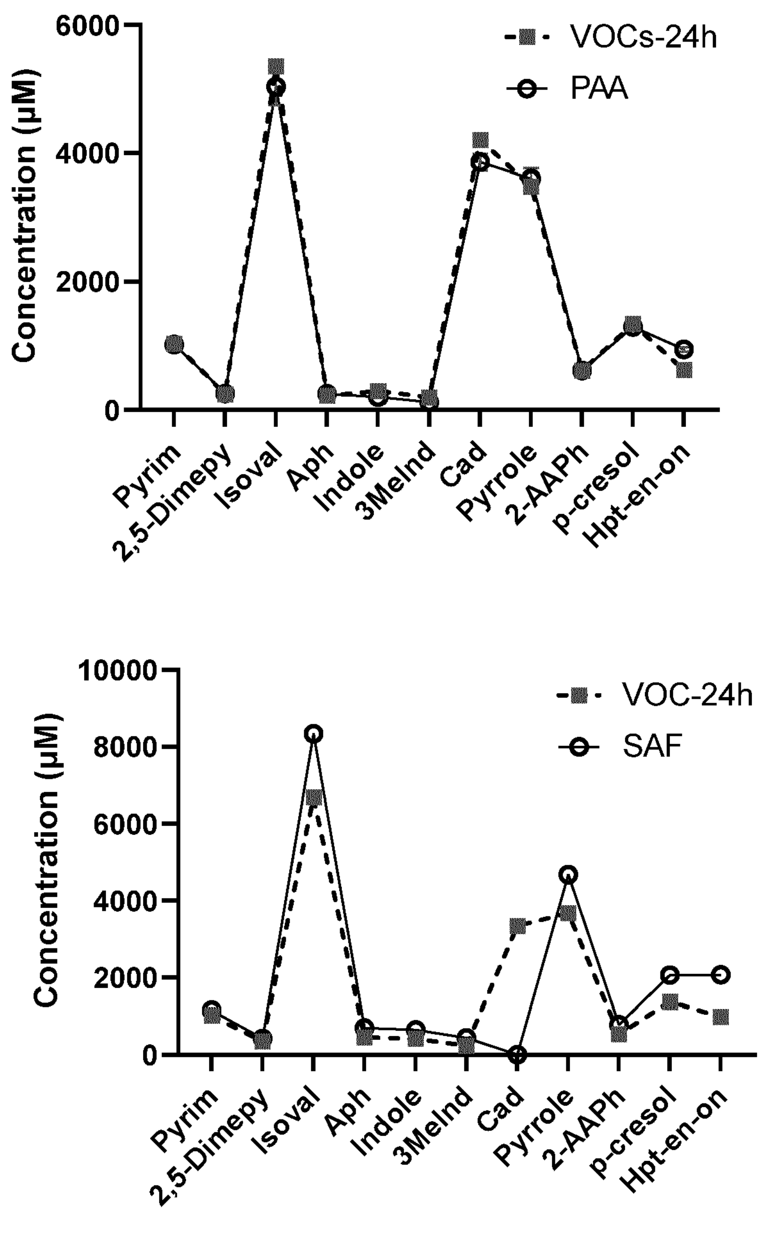
VOCs-24h
PAA
Concentration (µM)
6000
4000
2000
0
Pyrim
2,5-Dimepy
Isoval
Aph
Indole
3MeInd
Cad
Pyrrole
2-AAPh
p-cresol
Hpt-en-on
VOC-24h
SAF
Concentration (µM)
10000
8000
6000
4000
2000
0
Pyrim
2,5-Dimepy
Isoval
Aph
Indole
3MeInd
Cad
Pyrrole
2-AAPh
p-cresol
Hpt-en-on

Figure 4

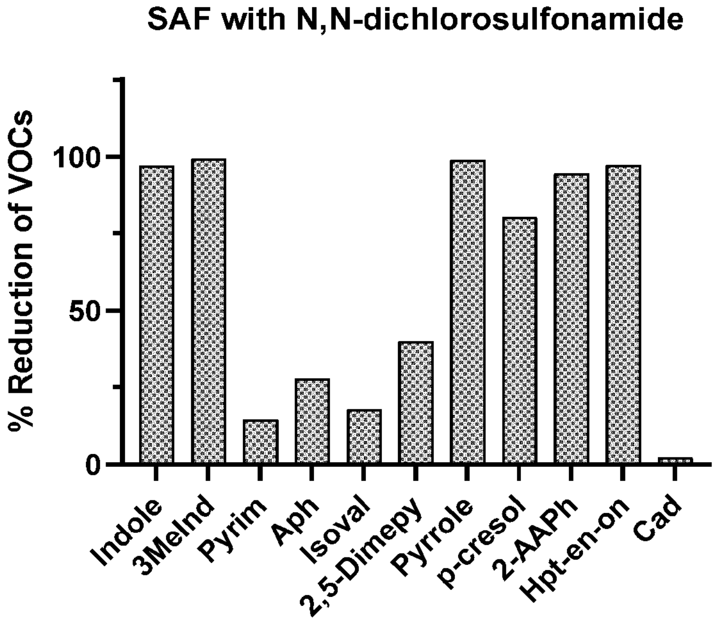
SAF with N,N-dichlorosulfonamide
% Reduction of VOCs
100
50
0
Indole
3MeInd
Pyrim
Aph
Isoval
2,5-Dimepy
Pyrrole
p-cresol
2-AAPh
Hpt-en-on
Cad

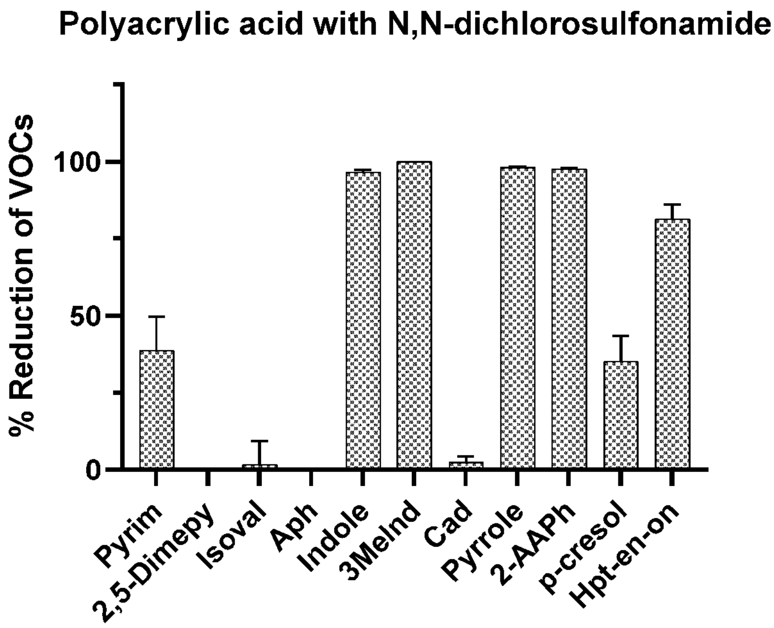
Polyacrylic acid with N,N-dichlorosulfonamide
% Reduction of VOCs
100
50
0
Pyrim
2,5-Dimepy
Isoval
Aph
Indole
3MeInd
Cad
Pyrrole
2-AAPh
p-cresol
Hpt-en-on

Figure 5

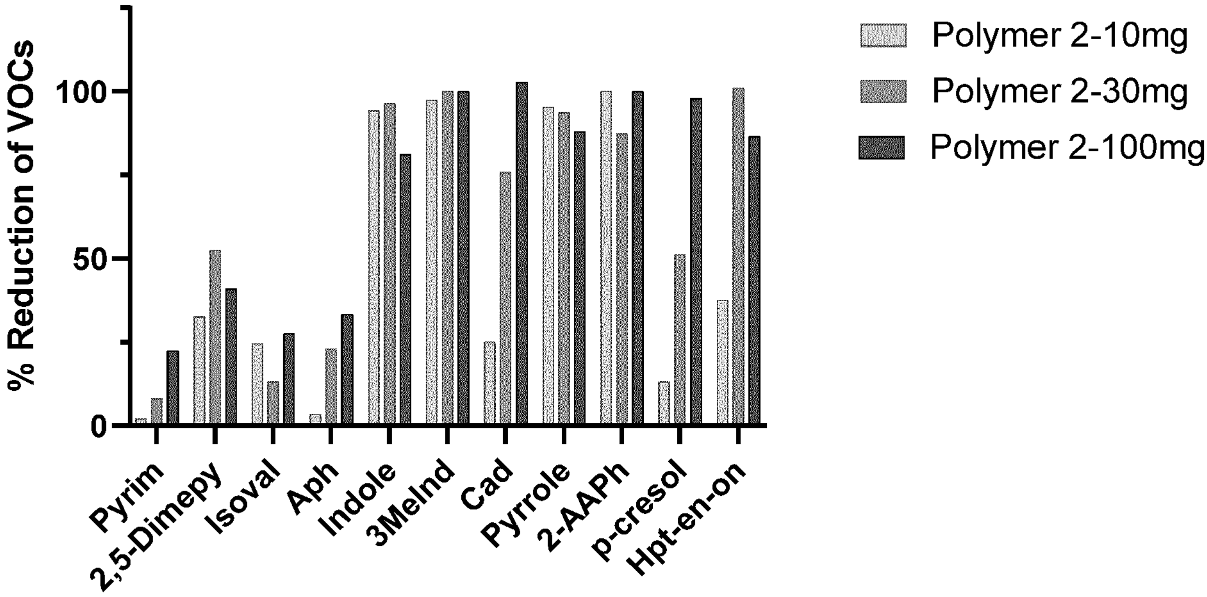


SAF modified with N-Chlorosulfonamide

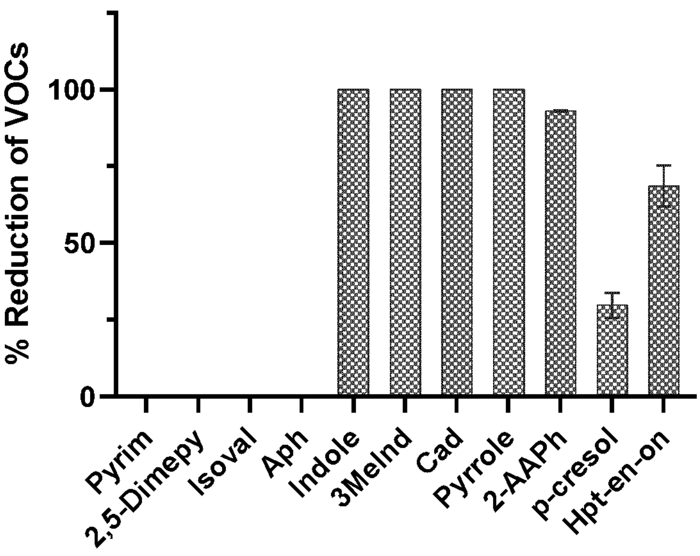

Figure 6

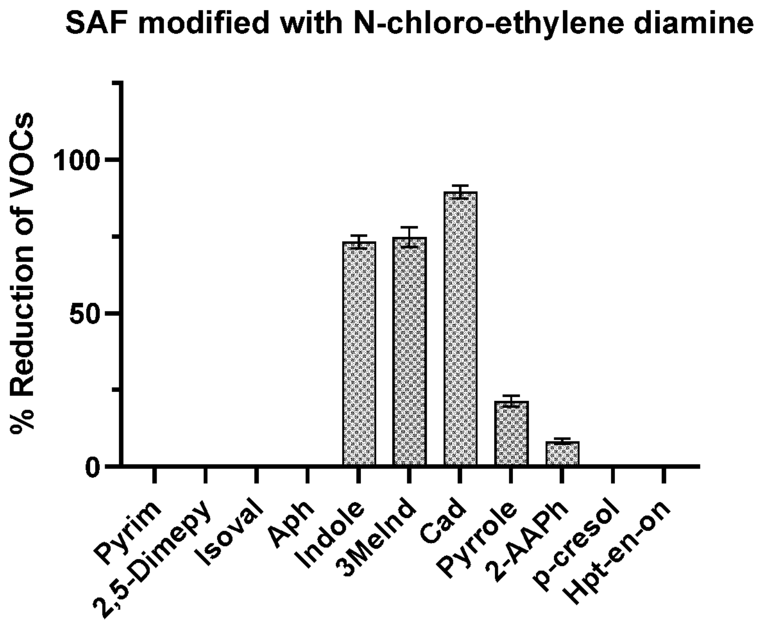
SAF modified with N-chloro-ethylene diamine
% Reduction of VOCs
100
50
0
Pyrim
2,5-Dimepy
Isoval
Aph
Indole
3MeInd
Cad
Pyrrole
2-AAPh
p-cresol
Hpt-en-on

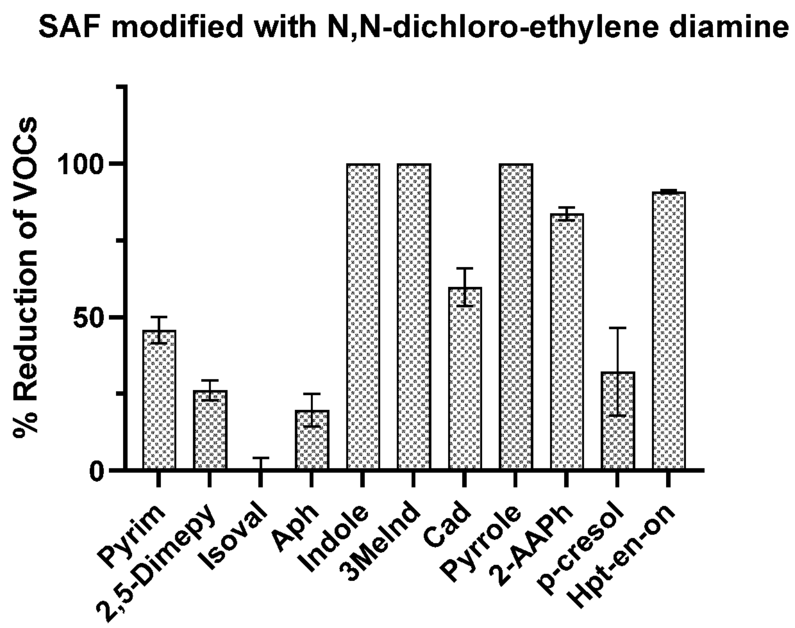
SAF modified with N,N-dichloro-ethylene diamine
% Reduction of VOCs
100
50
0
Pyrim
2,5-Dimepy
Isoval
Aph
Indole
3MeInd
Cad
Pyrrole
2-AAPh
p-cresol
Hpt-en-on

Figure 7

SAF modified with N,N-dichloro-diaminohexane

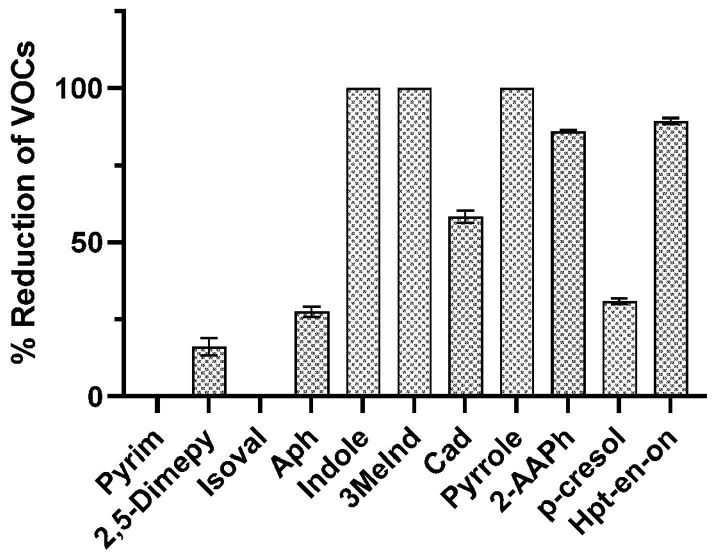


SAF modified with N-Chloro-diaminohexane

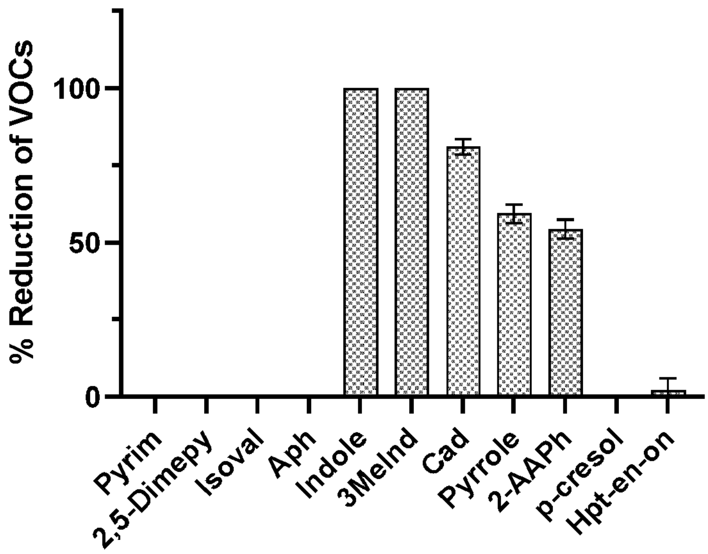

**Figure 8**

| **Materials** (chlorine content in %) | **% Reduction of VOCs** | | | | | | | | | | |
|---|---|---|---|---|---|---|---|---|---|---|---|
| | Pyrim | 2,5-Dimepy | Isoval | Aph | Indole | 3MeInd | Cad | Pyrrole | 2-AAPh | p-cresol | Hpt-en-on |
| Polymer 2 (4%) 100 mg | 14.69 | 40.02 | 17.96 | 27.77 | 97.21 | 99.48 | 2.22 | 99.15 | 94.45 | 80.48 | 97.45 |
| Polymer 2 (12-16%) 10 mg | 2.01 | 32.70 | 24.53 | 3.48 | 94.19 | 97.39 | 25.027 | 95.29 | 100 | 13.15 | 37.50 |
| Polymer 2 (12-16%) 30 mg | 8.13 | 52.59 | 13.17 | 23.02 | 96.38 | 100 | 75.75 | 93.62 | 87.38 | 51.16 | 100 |
| Polymer 2 (12-16%) 100 mg | 22. 42 | 40.84 | 27.65 | 33.43 | 81.19 | 100 | 100 | 87.95 | 100 | 97.88 | 86.53 |

**Figure 9**

| **Materials** (chlorine content in %) | **% Reduction of VOCs** | | | | | | | | | | |
|---|---|---|---|---|---|---|---|---|---|---|---|
| | Pyrim | 2,5-Dimepy | Isoval | Aph | Indole | 3MeInd | Cad | Pyrrole | 2-AAPh | p-cresol | Hpt-en-on |
| Polymer 5 (~0.3 - 0.6 %) | 0 | 0 | 0 | 0 | 73.25 | 74.7 | 89.5 | 21.4 | 8.3 | 0 | 0 |
| Polymer 4 (~8%) | 45.9 | 26.1 | 0 | 19.7 | 100 | 100 | 59.7 | 100 | 83.7 | 32.2 | 90.85 |
| Polymer 7 (~1%) | 0 | 0 | 0 | 0 | 100 | 100 | 80.9 | 59.3 | 54.4 | 0 | 0 |
| Polymer 6 (~8%) | 0 | 16.1 | 0 | 27.5 | 100 | 100 | 58.36 | 100 | 86.12 | 30.93 | 89.3 |
| Polymer 3 (~2%) | 0 | 0 | 0 | 0 | 100 | 100 | 100 | 100 | 92.9 | 29.7 | 68.5 |
| Polymer 2 (~14%) | 22.4 | 40.84 | 27.65 | 33.43 | 81.19 | 100 | 100 | 87.95 | 100 | 97.88 | 86.53 |

Europäisches Patentamt
European Patent Office
Office européen des brevets

# EUROPEAN SEARCH REPORT

Application Number
EP 21 20 2928

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/008608 A1 (PARKHURST STEPHEN L [US] ET AL) 13 January 2005 (2005-01-13)<br>* page 1, paragraph 9-10 *<br>* page 2, paragraph 18-21 *<br>* page 3, paragraph 26 *<br>* page 7, paragraph 38 *<br>* page 10, paragraph 109 - page 11, paragraph 111 *<br>* claims *<br>----- | 1-15 | INV.<br>A61L15/46<br>D06M13/265<br>A61L15/24<br>C08L33/08 |
| A | WO 2016/130588 A1 (REM BRANDS INC [US]) 18 August 2016 (2016-08-18)<br>* page 4, paragraph 12 - page 5, paragraph 15 *<br>* page 10, paragraph 42 - page 15, paragraph 61 *<br>* page 18, paragraph 76-77 *<br>* claims *<br>----- | 1-15 | |
| A,D | US 8 425 890 B2 (SCHNEIDER DAVID J [US]; SCHNEIDER CHARLES A [US]) 23 April 2013 (2013-04-23)<br>* page 1, paragraph 5 - page 2, paragraph 30 *<br>* page 3, paragraphs 41-44, 48 *<br>* claims *<br>----- | 1-15 | TECHNICAL FIELDS SEARCHED (IPC)<br>A61L<br>D06Q<br>D06M<br>C08F<br>C08L |
| A | US 2007/264520 A1 (WOOD WILLARD E [US] ET AL) 15 November 2007 (2007-11-15)<br>* page 2, paragraph 17 *<br>* page 9, paragraph 77-85 *<br>* page 12, paragraph 131 *<br>* page 28, paragraph 269-270 *<br>* page 30, paragraph 290 *<br>* claims *<br>----- | 1-15 | |

1 The present search report has been drawn up for all claims

| Place of search | Date of completion of the search | Examiner |
|---|---|---|
| The Hague | 16 March 2022 | Van den Bulcke, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 20 2928

This annex lists the patent family members relating to the patent documents cited in the above-mentioned European search report. The members are as contained in the European Patent Office EDP file on
The European Patent Office is in no way liable for these particulars which are merely given for the purpose of information.

16-03-2022

| Patent document cited in search report | Publication date | Patent family member(s) | Publication date |
|---|---|---|---|
| US 2005008608 A1 | 13-01-2005 | US 2005008608 A1 | 13-01-2005 |
| | | WO 2005006862 A2 | 27-01-2005 |
| WO 2016130588 A1 | 18-08-2016 | BR 112017015903 A2 | 10-04-2018 |
| | | CA 2974980 A1 | 18-08-2016 |
| | | CN 107405246 A | 28-11-2017 |
| | | EP 3256088 A1 | 20-12-2017 |
| | | JP 6820108 B2 | 27-01-2021 |
| | | JP 2018510039 A | 12-04-2018 |
| | | JP 2021035495 A | 04-03-2021 |
| | | WO 2016130588 A1 | 18-08-2016 |
| US 8425890 B2 | 23-04-2013 | CN 102387706 A | 21-03-2012 |
| | | CN 106880866 A | 23-06-2017 |
| | | EP 2400995 A2 | 04-01-2012 |
| | | EP 2946794 A1 | 25-11-2015 |
| | | JP 5636005 B2 | 03-12-2014 |
| | | JP 2012518491 A | 16-08-2012 |
| | | US 2010215612 A1 | 26-08-2010 |
| | | US 2013236414 A1 | 12-09-2013 |
| | | WO 2010099110 A2 | 02-09-2010 |
| US 2007264520 A1 | 15-11-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8425890 B
- US 8425890 A


### Non-patent literature cited in the description


- **A. AKHMETOVA et al.** A Comprehensive Review of Topical Odor-Controlling Treatment Options for Chronic Wounds. *J Wound Ostomy Continence Nurs,* 2016, vol. 43 (6), 598-609
- *Pure Appl. Chem.,* 2004, vol. 76 (4), 889-906